(19) Europäisches Patentamt
European Patent Office
Office européen des brevets

(11) **EP 3 861 932 B1**

(12) **EUROPÄISCHE PATENTSCHRIFT**

(45) Veröffentlichungstag und Bekanntmachung des Hinweises auf die Patenterteilung:
**10.07.2024 Patentblatt 2024/28**

(21) Anmeldenummer: **21020041.6**

(22) Anmeldetag: **02.02.2021**

(51) Internationale Patentklassifikation (IPC):
*A61B 5/08* (2006.01)   *A61B 5/085* (2006.01)
*A61M 16/00* (2006.01)   *A61B 5/091* (2006.01)
*A61B 5/0536* (2021.01)   *A61B 5/00* (2006.01)

(52) Gemeinsame Patentklassifikation (CPC):
**A61B 5/0809; A61B 5/0536; A61B 5/085; A61B 5/091; A61B 5/7425; A61B 5/743; A61M 16/00;** A61M 2016/0027; A61M 2016/0033; A61M 2230/46; A61M 2230/65

(54) **VERFAHREN UND VORRICHTUNG ZUR BESTIMMUNG EINER REGIONALEN COMPLIANCE EINER LUNGE BEI SPONTANATMUNG**

METHOD AND DEVICE FOR DETERMINING REGIONAL COMPLIANCE OF A LUNG IN SPONTANEOUS BREATHING

PROCÉDÉ ET DISPOSITIF DE DÉTERMINATION D'UNE CONFORMITÉ RÉGIONALE D'UN POUMON LORS DE LA RESPIRATION SPONTANÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.02.2020 DE 102020103145**

(43) Veröffentlichungstag der Anmeldung:
**11.08.2021 Patentblatt 2021/32**

(73) Patentinhaber: **Löwenstein Medical Technology S.A.**
**2557 Luxembourg (LU)**

(72) Erfinder: **Kremeier, Peter**
**76149 Karlsruhe (DE)**

(74) Vertreter: **Marx, Thomas**
**Löwenstein Medical Technology GmbH + Co. KG**
**IP Management**
**Kronsaalsweg 40**
**22525 Hamburg (DE)**

(56) Entgegenhaltungen:
**DE-A1- 102013 203 177   US-A1- 2018 078 168**

- **TOBIAS H BECHER ET AL: "Assessment of respiratory system compliance with electrical impedance tomography using a positive end-expiratory pressure wave maneuver during pressure support ventilation: a pilot clinical study", CRITICAL CARE, BIOMED CENTRAL LTD LONDON, GB, vol. 18, no. 6, 10 December 2014 (2014-12-10), pages 679, XP021210348, ISSN: 1364-8535, DOI: 10.1186/S13054-014-0679-6**
- **ZHAO ZHANQI ET AL: "PEEP titration guided by ventilation homogeneity: a feasibility study using electrical impedance tomography", CRITICAL CARE, BIOMED CENTRAL LTD LONDON, GB, vol. 14, no. 1, 30 January 2010 (2010-01-30), pages R8, XP021070816, ISSN: 1364-8535**

**Beschreibung**

Die Erfindung betrifft ein Verfahren zur Bestimmung einer regionalen Compliance einer Lunge bei Spontanatmung. Die Erfindung betrifft ferner ein System zur Bestimmung einer regionalen Compliance einer Lunge bei Spontanatmung. Außerdem betrifft die Erfindung ein Beatmungsgerät umfassend das System zur Bestimmung der regionalen Compliance.

Unter dem Begriff Compliance wird in der Physiologie die Dehnbarkeit von Atmungs- und Gefäßsystemen verstanden. Anhand der Compliance der Lunge kann ein Zustand von Lungengewebe beurteilt werden. Zur Ermittlung der Compliance der Lunge wird eine Volumenerhöhung der Lunge mit einem Füllungsdruck in Relation gesetzt. Je höher die Compliance ist, umso stärker dehnt sich das Lungengewebe aus, wenn die Lunge mit einem gewissen Füllungsdruck beaufschlagt wird. Lungengewebe mit einer niedrigen Compliance ist hingegen vergleichsweise steif. Eine niedrige Compliance der Lunge kann durch diverse Erkrankungen bedingt sein. Auf Grundlage der Compliance können beispielsweise Parameter von Beatmungsgeräten eingestellt werden, um einen möglichst lungenschonenden Betrieb bei einer künstlichen Beatmung mittels des Beatmungsgeräts zu ermöglichen. Unter mandatorischer Beatmung kann die Compliance einer Lunge vergleichsweise einfach gemessen werden. Bei der Spontanatmung ist dies aufgrund der elastischen Rückstellkräfte der Lunge und der Zwerchfellaktivität hingegen nicht der Fall.

Mittels EIT (Elektro-Impedanz-Tomographie) können räumlich differenzierte Informationen zum Zustand einer Lunge gewonnen werden. Die EIT ist ein nicht-invasives Verfahren, bei dem elektrische Leitfähigkeiten am menschlichen Körper gemessen werden. Dabei werden EIT-Pixel aufgenommen, wobei jedes EIT-Pixel eine elektrische Leitfähigkeit des menschlichen Körpers in einem bestimmten Körperareal wiedergibt. Wird dieses Verfahren im Bereich der Lunge durchgeführt, dann ergeben sich für die EIT-Pixel unterschiedliche Werte in Abhängigkeit von einem Zustand der Lunge. Unterschiede in der elektrischen Leitfähigkeit entstehen insbesondere in Abhängigkeit von der Belüftung der Lunge. Daraus lassen sich beispielsweise Rückschlüsse auf den Zustand der Lungenbläschen in bestimmten Bereichen der Lunge ziehen. Die EIT kann direkt am Bett des Patienten durchgeführt werden. Zu Details der EIT wird auf das Dokument DE 10 2013 203177 A1 verwiesen.

Der Begriff der regionalen Compliance beschreibt die Compliance in bestimmten Bereichen der Lunge. Die regionale Compliance vermittelt klinisch relevante Informationen zur Abschätzung der Inhomogenität der Lunge. Sie kann bei der Einschätzung von Auswirkungen auf Veränderungen an Beatmungsparametern und bei der Durchführung von Beatmungsmanövern hilfreich sein.

Der vorliegenden Erfindung liegt vor diesem Hintergrund die Aufgabe zugrunde, ein Verfahren zur Bestimmung einer regionalen Compliance einer Lunge beispielsweise bei Spontanatmung bereitzustellen. Es ist eine weitere Aufgabe, ein System zur Bestimmung einer regionalen Compliance einer Lunge beispielsweise bei Spontanatmung bereitzustellen. Es ist eine weitere Aufgabe, ein Beatmungsgerät bereitzustellen, das es erlaubt, eine regionale Compliance einer Lunge beispielsweise bei Spontanatmung zu ermitteln.

Die Aufgaben werden gelöst, zum einen durch ein Verfahren zur Bestimmung einer regionalen Compliance einer Lunge beispielsweise bei Spontanatmung nach Anspruch 1 und/oder 2, ferner durch ein System zur Bestimmung einer regionalen Compliance einer Lunge bei Spontanatmung nach Anspruch 10, sowie durch ein Beatmungsgerät nach Anspruch 15 und eine Einrichtung zur Elektro-Impedanz-Tomographie nach Anspruch 21.

Die Unteransprüche betreffen verschiedene voneinander unabhängige, vorteilhafte Weiterbildungen der vorliegenden Erfindung, deren Merkmale vom Fachmann im Rahmen des technisch Sinnvollen frei miteinander kombiniert werden können. Dies gilt insbesondere auch über die Grenzen der verschiedenen Anspruchskategorien hinaus.

Gemäß einem ersten Aspekt der Erfindung wird ein Verfahren zur Bestimmung einer regionalen Compliance $C.REG_i$ einer Lunge bei Spontanatmung vorgeschlagen. Bei dem Verfahren erfolgt eine Bestimmung eines Lungendehnbarkeitswerts C.M durch eine Auslösung eines Inspirationsmanövers, sowie entweder durch eine Messung einer Compliance der gesamten Lunge oder durch eine Messung eines Surrogates für die Compliance der gesamten Lunge. Bei dem Verfahren erfolgt ferner eine Erfassung einer elektrischen Impedanzverteilung entlang wenigstens eines zweidimensionalen Schnitts durch einen menschlichen Thorax mittels einer Einrichtung zur Elektro-Impedanz-Tomographie. Bei dem Verfahren erfolgt ferner eine Unterteilung der erfassten elektrischen Impedanzverteilung zu verschiedenen Zeitpunkten in eine Vielzahl von EIT-Pixeln. Bei dem Verfahren erfolgt ferner eine Bestimmung einer endexspiratorischen elektrischen Impedanz Z.EXSP jedes der EIT-Pixel sowie einer endinspiratorischen elektrischen Impedanz Z.INSP jedes der EIT-Pixel. Bei dem Verfahren erfolgt ferner eine Bestimmung einer Impedanzdifferenz ΔZ jedes der EIT-Pixel, indem eine Differenz zwischen der endinspiratorischen elektrischen Impedanz Z.INSP und der endexspiratorischen elektrischen Impedanz Z.EXSP gebildet wird. Bevorzugt wird hierzu die endexspiratorische elektrische Impedanz Z.EXSP von der endinspiratorischen elektrischen Impedanz Z.INSP subtrahiert. Bei dem Verfahren erfolgt ferner eine Bereitstellung mehrerer Impedanz-Wertebereiche und eine Zuordnung der EIT-Pixel zu den Impedanz-Wertebereichen in Abhängigkeit von der jeweiligen Impedanzdifferenz ΔZ der EIT-Pixel. Bei dem Verfahren erfolgt ferner eine Bestimmung der regionalen Compliance $C.REG_i$ für spezifische EIT-Pixel, die einem Impedanz-Wertebereich i zugeordnet sind, in Abhängigkeit von einer Häufigkeitsverteilung aller EIT-Pixel auf die Impedanz-Wertebereiche und in Abhängigkeit von dem Lungendehnbarkeitswert C.M.

Zur Bestimmung des Lungendehnbarkeitswerts C.M wird ein Inspirationsmanöver ausgelöst. Darunter ist zu verstehen, dass beim Einatmen gezielt Einfluss auf die Lunge genommen wird. Die Einflussnahme kann erfindungsgemäß mittels eines Beatmungsgeräts erfolgen, ganz besonders bevorzugt mittels eines Beatmungsgeräts zur druckunterstützenden Beatmung. Der Lungendehnbarkeitswert C.M kann bestimmt werden, indem eine Compliance der gesamten Lunge ermittelt wird. Dazu werden bevorzugt ein endexspiratorischer Druck, ein endinspiratorischer Druck und ein Tidalvolumen gemessen, aus denen die Compliance der gesamten Lunge berechnet wird. Der endexspiratorische Druck und/oder der endinspiratorische Druck werden bevorzugt bei Okklusion der Atemwege gemessen. Es ist auch möglich, dass eine Messung eines Surrogates für die Compliance der gesamten Lunge erfolgt, um den Lungendehnbarkeitswert zu bestimmen. Dabei können unterschiedliche Verfahren zum Einsatz kommen, die dazu geeignet sind, den Lungendehnbarkeitswert hinreichend genau zu bestimmen.

Eine endinspiratorische elektrische Impedanz Z.INSP jedes der EIT-Pixel sowie eine endexspiratorische elektrische Impedanz Z.EXSP jedes der EIT-Pixel werden bestimmt. Dies erfolgt bevorzugt zu Beginn beziehungsweise am Ende eines Inspirationsvorgangs der Lunge, bei dem auch das vorab beschriebene Inspirationsmanöver ausgelöst wird. Es ist alternativ jedoch auch möglich, dass Z.INSP und Z.EXSP während vorhergehender oder nachfolgender Atembewegungen bestimmt werden. Es ist vorteilhaft, wenn ab der Messung der exspiratorischen elektrischen Impedanz bis zur Messung der endinspiratorischen elektrischen Impedanz eine elektrische Impedanz für jedes EIT-Pixel kontinuierlich erfasst wird.

Die Impedanzdifferenz $\Delta Z$ für jedes EIT-Pixel wird bestimmt, indem die Differenz zwischen Z.INSP und Z.EXSP gebildet wird. Die jeweiligen Impedanzdifferenzen $\Delta Z$ der EIT-Pixel sind Indikatoren für einen Zustand der Lunge in unterschiedlichen Lungenregionen, was einen Rückschluss auf die regionale Compliance $C.REG_i$ erlaubt. Die EIT-Pixel werden den Impedanz-Wertebereichen in Abhängigkeit von ihrem jeweiligen Wert $\Delta Z$ zugeordnet. In Abhängigkeit von einer Häufigkeitsverteilung aller EIT-Pixel auf die Impedanz-Wertebereiche und in Abhängigkeit von dem Lungendehnbarkeitswert C.M kann nun die regionale Compliance $C.REG_i$ bestimmt werden. Dabei wird in vorteilhafter Weise ausgenutzt, dass zwischen den Impedanzdifferenzen $\Delta Z$ der EIT-Pixel und der Ausprägung der Compliance in unterschiedlichen Bereichen der Lunge ein Zusammenhang besteht. Die regionale Compliance $C.REG_i$ bezieht sich auf spezifische EIT-Pixel, die einem Impedanz-Wertebereich i zugeordnet sind, wobei i eine laufende Nummer ist, die unterschiedliche Impedanz-Wertebereiche voneinander unterscheidet. In Bereichen der Lunge, die von diesen spezifischen EIT-Pixeln erfasst sind, liegt folglich die sogenannte regionale Compliance $C.REG_i$ vor.

Gegeben seien verschiedene Impedanz-Wertebereiche der Anzahl m. Dann können auch m regionale Compliances $C.REG_i$ innerhalb der Lunge bestimmt werden mit den Laufnummern i = 1, 2, ..., m. Diese regionalen Compliances können erfindungsgemäß gesammelt und ausgegeben werden, beispielsweise auf einem Anzeigemittel oder in einem digitalen Datenformat. Die regionalen Compliances können erfindungsgemäß zu verschiedenen Zeitpunkten ermittelt werden, sodass ein zeitlicher Verlauf der regionalen Compliances in unterschiedlichen Lungenbereichen nachvollzogen werden kann.

Die Bestimmung der regionalen Compliance $C.REG_i$ erfolgt vorzugweise bei einer druckunterstützenden Beatmung der Lunge. Unter dem Begriff der druckunterstützenden Beatmung der Lunge ist zu verstehen, dass die Lungenfunktion beim spontanen Einatmen durch Beaufschlagung mit einem zusätzlichen Druck unterstützt wird, der fest vorgegeben wurde, sich am einem Zielvolumen orientiert oder proportional zu den Strömungswiederständen und dem inspiratorischen Volumen realisiert wird. Es wird also eine Hilfestellung bei der Spontanatmung geleistet. Atemfrequenz und Atemtiefe können meist vom Patienten selbst bestimmt werden. Die druckunterstützende Beatmung erfolgt mittels eines Beatmungsgeräts. Es wird in der Regel ein gewünschter Druck voreingestellt. Diese Form der Beatmung kann beispielsweise zur Abgewöhnung von Beatmungsgeräten verwendet werden.

Es ist bevorzugt, wenn das Inspirationsmanöver ein endinspiratorisches Holdmanöver ist, das bei der druckunterstützenden Beatmung der Lunge ausgeführt wird. Unter einem endinspiratorischen Holdmanöver ist ein Beatmungsmanöver zu verstehen, bei dem gegen Ende oder am Ende des Einatmens der Luftfluss unterbrochen wird. Gemäß Ausführungsformen der Erfindung kann während des endinspiratorischen Holdmanövers ein endinspiratorischer Druck gemessen werden, der zur Bestimmung des Lungendehnbarkeitswerts C.M genutzt wird. Erfindungsgemäß kann während des endinspiratorischen Holdmanövers ferner die endinspiratorische elektrische Impedanz Z.INSP gemessen werden. Es kann erfindungsgemäß vorgesehen sein, dass das endinspiratorische Holdmanöver in Abhängigkeit von einem gemessenen Druck, in Abhängigkeit von einer Größe eines Volumenstroms in die Lunge, in Abhängigkeit von einem der Lunge zugeführten Volumen und/oder in Abhängigkeit von einem sonstigen Parameter ausgelöst wird. Ferner ist es möglich, dass ein Zeitpunkt, zu dem das endinspiratorische Manöver ausgelöst wird, manuell bestimmt wird. Weitere Informationen zur Bestimmung der Compliance mittels endinspiratorischer Holdmanöver können aus dem Beitrag "End-inspiratory airway occlusion: a method to assess the pressure developed by inspiratory muscles in patients with acute lung injury undergoing pressure support", Foti et al., American Journal of Respiratory and Critical Care Medicine, Volume 156, No. 4 Pt. 1, S. 1210-1216, 1997, sowie aus dem Beitrag "Driving Pressure Is Associated with Outcome during Assisted Ventilation in Acute Respiratory Distress Syndrome", Bellani et al., Anesthesiology, Volume 131, No. 3, S. 594-604, 2019, entnommen werden.

Es kann erfindungsgemäß vorgesehen sein, dass der Lungendehnbarkeitswert C.M gemäß

$$C.M = \frac{TV}{P.PLATEAU - EEP}$$

bestimmt wird, wobei TV ein Tidalvolumen der Lunge ist, wobei P.PLATEAU ein endinspiratorischer Plateaudruck ist, der sich bei dem endinspiratorischen Holdmanöver einstellt, und wobei EEP ein endexspiratorischer Druck ist. Das Tidalvolumen TV bezeichnet ein Lungenvolumen, das vorzugsweise bei der Inspiration bestimmt wird. Es kann beispielsweise mittels eines Durchflusssensors bestimmt werden. Der endexspiratorische Druck wird vorzugsweise vor Beginn der Inspiration bestimmt. Der endinspiratorische Plateaudruck stellt sich nach Auslösung des endinspiratorischen Holdmanövers nach kurzer Zeit unter Okklusion der Atemwege ein. Er liegt in der Regel über einem Spitzendruck der Spontanatmung. Bevorzugt wird die endinspiratorische elektrische Impedanz Z.INSP genau dann bestimmt, wenn sich der endinspiratorische Plateaudruck P.PLATEAU eingestellt hat.

Gemäß einer vorteilhaften Ausführungsform der Erfindung wird die regionale Compliance C.REGi gemäß

$$C.REG_i = \frac{W_i.UB}{\sum_{n=1}^{m}(NPX_n * W_n.UB)} * C.M * \sum_{n=1}^{m} NPX_n$$

bestimmt, wobei die EIT-Pixel mehreren Impedanz-Wertebereichen der Anzahl m zugeordnet sind, wobei $NPX_n$ eine Anzahl von EIT-Pixeln ist, die einem n-ten Impedanz-Wertebereich der m Impedanz-Wertebereiche zugeordnet sind, wobei $W_i.UB$ eine Obergrenze des Impedanz-Wertebereichs i ist, und wobei $W_n.UB$ eine Obergrenze des n-ten Impedanz-Wertebereichs der m Impedanz-Wertebereiche ist.

Es sind m Impedanz-Wertebereiche $W_1$, $W_2$, ..., $W_m$ gegeben. Der n-te Impedanz-Wertebereich $W_n$ weist eine Obergrenze (so genannte "upper bound") $W_n.UB$ auf. Der Impedanz-Wertebereich i, der mit $W_i$ bezeichnet werden soll, wobei $1 \leq i \leq m$ gilt, weist eine Obergrenze ("upper bound") $W_i.UB$ auf. Mittels der aufgeführten Formel wird der Lungendehnbarkeitswert C.M in Bezug auf die Impedanz-Wertebereiche und die diesen zuzuordnenden EIT-Pixel so gewichtet, dass sich die regionalen Compliances $C.REG_i$ für unterschiedliche Impedanz-Wertebereiche i ergeben.

Es kann erfindungsgemäß vorgesehen sein, dass die Impedanz-Wertebereiche ermittelt werden, indem eine minimale ermittelte Impedanzdifferenz $\Delta Z_{min}$ von einer maximalen ermittelten Impedanzdifferenz $\Delta Z_{max}$ subtrahiert wird und ein sich ergebender Zahlenbereich in gleich große Abschnitte unterteilt wird, wobei die Abschnitte die Impedanz-Wertebereiche bilden. Die minimale Impedanzdifferenz $\Delta Z_{min}$ und die maximale Impedanzdifferenz $\Delta Z_{max}$ werden bestimmt, um für die zu betrachtenden Impedanz-Wertebereiche eine Gesamtuntergrenze und eine Gesamtobergrenze zu ermitteln. Ein sich ergebender Zahlenbereich wird in gleich große Abschnitte unterteilt. Gemäß alternativen Ausführungsformen ist es jedoch auch möglich, den Zahlenbereich in unterschiedlich große Abschnitte zu unterteilen, sodass sich unterschiedlich große Impedanz-Wertebereiche ergeben.

Vorzugsweise wird einem Impedanz-Wertebereich $W_n$ mit einer Untergrenze $W_n.LB$ und mit einer Obergrenze $W_n.UB$ ein EIT-Pixel mit dem Differenzwert $\Delta Z$ genau dann zugeordnet, wenn folgendes gilt:

$$W_n.LB \leq \Delta Z < W_n.UB$$

oder

$$W_n.LB < \Delta Z \leq W_n.UB.$$

Jeder Impedanz-Wertebereich weist eine Untergrenze $W_n.LB$ und eine Obergrenze $W_n.UB$ auf. Es können unterschiedliche Zuordnungsvorschriften zur Anwendung kommen.

Es kann erfindungsgemäß vorgesehen sein, dass bei dem Verfahren ein Anteil $A_i$ eines Lungenvolumens, in dem die regionale Compliance $C.REG_i$ vorliegt, gemäß

$$A_i = \frac{NPX_i}{\sum_{n=1}^{m} NPX_n}$$

bestimmt wird, wobei die EIT-Pixel m Impedanz-Wertebereichen zugeordnet sind, wobei $NPX_i$ eine Anzahl der spezifischen EIT-Pixel ist und wobei $NPX_n$ eine Anzahl von EIT-Pixeln ist, die einem n-ten Impedanz-Wertebereich der m Impedanz-Wertebereiche zugeordnet sind. Auf diese Weise wird ermittelt, welcher Anteil der EIT-Pixel die regionale Compliance $C.REG_i$ aufweist.

Gemäß einem weiteren Aspekt der Erfindung wird ein System zur Bestimmung einer regionalen Compliance $C.REG_i$ einer Lunge bei Spontanatmung vorgeschlagen, wobei das System eine Einrichtung zur Elektro-Impedanz-Tomographie und Mittel zur Bestimmung eines Lungendehnbarkeitswerts C.M durch eine Messung einer Compliance der Lunge oder durch eine Messung eines Surrogates für die Compliance der Lunge aufweist, und wobei das System zur Durchführung des vorangehend beschriebenen Verfahrens eingerichtet ist. Ein solches System kann die regionale Compliance bestimmen. Gemäß möglichen Ausführungsformen der Erfindung kann es sich um ein eigenständiges Gerät handeln. Insbesondere kann eine Einrichtung zur Elektro-Impedanz-Tomographie als ein eigenständiges Gerät ausgebildet sein, welches beispielsweise mit einem Beatmungsgerät interagieren und Daten austauschen kann. Dazu sind das Beatmungsgerät und die Einrichtung zur Elektro-Impedanz-Tomographie über zumindest eine Schnittstelle gekoppelt. Entsprechend weisen das Beatmungsgerät und die Einrichtung zur Elektro-Impedanz-Tomographie jeweils zumindest eine Schnittstelle auf.

Die Mittel zur Bestimmung eines Lungendehnbarkeitswerts C.M können dann in dem Beatmungsgerät oder in der Einrichtung zur Elektro-Impedanz-Tomographie ausgebildet sein.

Es ist jedoch auch möglich, dass das System in eine andere Vorrichtung integriert ist und/oder dazu geeignet ist, zusammen mit einer anderen Vorrichtung verwendet zu werden. Die Mittel zur Bestimmung der Compliance umfassen bevorzugt einen Drucksensor, einen Durchflusssensor und/oder eine Atemgasquelle.

Bevorzugt weist das System zur Bestimmung der regionalen Compliance $C.REG_i$ eine Rechnereinheit und/oder ein Anzeigemittel auf. Die Rechnereinheit ist bevorzugt dazu eingerichtet, auf Grundlage von Ausgabewerten der Einrichtung zur Elektro-Impedanz-Tomographie, des Drucksensors und/oder des Durchflusssensors (des Beatmungsgerätes) die regionale Compliance $C.REG_i$ zu berechnen. Die Rechnereinheit kann erfindungsgemäß ferner dazu eingerichtet sein, einen Anteil $A_i$ eines Lungenvolumens, in dem die regionale Compliance $C.REG_i$ vorliegt, zu berechnen. Ferner kann die Rechnereinheit dazu eingerichtet sein, das Anzeigemittel dazu zu veranlassen, die regionale Compliance $C.REG_i$ und/oder den Anteil $A_i$ auszugeben. Die regionale Compliance $C.REG_i$ wird vorzugsweise in der Einheit ml/mbar ausgegeben. Der Anteil $A_i$ wird vorzugsweise als ein Prozentsatz ausgegeben.

Die Rechnereinheit ist beispielsweise als Teil des Beatmungsgerätes ausgebildet dazu eingerichtet, die erfindungsgemäßen Verfahrensschritte und die dazu notwendigen Berechnungen auszuführen. Die Rechnereinheit ist alternativ als Teil des Systems oder der Einrichtung zur Elektro-Impedanz-Tomographie ausgebildet dazu eingerichtet, die erfindungsgemäßen Verfahrensschritte und die dazu notwendigen Berechnungen auszuführen.

Das System kann erfindungsgemäß dazu eingerichtet sein, einen Lungendehnbarkeitswert C.M durch eine Auslösung eines Inspirationsmanövers, sowie entweder durch eine Messung einer Compliance der gesamten Lunge oder durch eine Messung eines Surrogates für die Compliance der gesamten Lunge zu bestimmen, eine elektrische Impedanzverteilung entlang wenigstens eines zweidimensionalen Schnitts durch einen menschlichen Thorax mittels einer Einrichtung zur Elektro-Impedanz-Tomographie zu erfassen, die erfasste elektrische Impedanzverteilung zu verschiedenen Zeitpunkten in eine Vielzahl von EIT-Pixeln zu unterteilen, eine endexspiratorische elektrische Impedanz Z.EXSP jedes der EIT-Pixel sowie eine endinspiratorische elektrische Impedanz Z.INSP jedes der EIT-Pixel zu bestimmen, eine Impedanzdifferenz $\Delta Z$ jedes der EIT-Pixel zu bestimmen, indem es eine Differenz zwischen der endinspiratorischen elektrischen Impedanz Z.INSP und der endexspiratorischen elektrischen Impedanz Z.EXSP bildet, mehrere Impedanz-Wertebereiche bereitzustellen und die EIT-Pixel den Impedanz-Wertebereichen in Abhängigkeit von der jeweiligen Impedanzdifferenz $\Delta Z$ der EIT-Pixel zuzuordnen, und die regionale Compliance $C.REG_i$ für spezifische EIT-Pixel, die einem Impedanz-Wertebereich i zugeordnet sind, in Abhängigkeit von einer Häufigkeitsverteilung aller EIT-Pixel auf die Impedanz-Wertebereiche und in Abhängigkeit von dem Lungendehnbarkeitswert C.M zu bestimmen.

Es kann ferner vorgesehen sein, dass das System dazu geeignet ist, die Bestimmung der regionalen Compliance $C.REG_i$ bei einer druckunterstützenden Beatmung der Lunge durchzuführen. Es ist ferner möglich, dass das Inspirationsmanöver, das das System auslöst, ein endinspiratorisches Holdmanöver ist, wobei das System dazu eingerichtet ist, das endinspiratorische Holdmanöver bei der druckunterstützenden Beatmung der Lunge auszuführen.

Bevorzugt ist das System dazu eingerichtet, den Lungendehnbarkeitswert C.M gemäß

$$C.M = \frac{TV}{P.PLATEAU - EEP}$$

zu bestimmen, wobei TV ein Tidalvolumen der Lunge ist, wobei P.PLATEAU ein endinspiratorischer Plateaudruck ist, der sich nach der Auslösung des endinspiratorischen Holdmanövers einstellt, und wobei EEP ein endexspiratorischer Druck ist.

Besonders bevorzugt ist das System dazu eingerichtet, die regionale Compliance $C.REG_i$ gemäß

$$C.REG_i = \frac{W_i.UB}{\sum_{n=1}^{m}(NPX_n * W_n.UB)} * C.M * \sum_{n=1}^{m}NPX_n$$

zu bestimmen, wobei die EIT-Pixel m Impedanz-Wertebereichen zugeordnet sind, wobei $NPX_n$ eine Anzahl von EIT-Pixeln ist, die einem n-ten Impedanz-Wertebereich der m Impedanz-Wertebereiche zugeordnet sind, wobei $W_i.UB$ eine Obergrenze des Impedanz-Wertebereichs i ist, und wobei $W_n.UB$ eine Obergrenze eines n-ten Impedanz-Wertebereichs der m Impedanz-Wertebereiche ist.

Es ist erfindungsgemäß möglich, dass das System dazu eingerichtet ist, die Impedanz-Wertebereiche zu ermitteln, indem es eine minimale ermittelte Impedanzdifferenz $\Delta Z_{min}$ von einer maximalen ermittelten Impedanzdifferenz $\Delta Z_{max}$ subtrahiert und einen sich ergebenden Zahlenbereich in gleich große Abschnitte unterteilt, wobei die Abschnitte die Impedanz-Wertebereiche bilden. Ferner kann das System so eingerichtet sein, dass es einem Impedanz-Wertebereich $W_n$ mit einer Untergrenze $W_n.LB$ und mit einer Obergrenze $W_n.UB$ ein EIT-Pixel mit dem Differenzwert $\Delta Z$ genau dann zuordnet, wenn folgendes gilt:

$$W_n.LB \leq \Delta Z < W_n.UB$$

oder

$$W_n.LB < \Delta Z \leq W_n.UB.$$

Eine vorteilhafte Ausführungsform des Systems ist dazu eingerichtet, einen Anteil A eines Lungenvolumens, in dem die regionale Compliance $C.REG_i$ vorliegt, gemäß

$$A = \frac{NPX_i}{\sum_{n=1}^{m}NPX_n}$$

zu bestimmen, wobei die EIT-Pixel m Impedanz-Wertebereichen zugeordnet sind, wobei $NPX_i$ eine Anzahl der spezifischen EIT-Pixel ist und wobei $NPX_n$ eine Anzahl von EIT-Pixeln ist, die einem n-ten Impedanz-Wertebereich der m Impedanz-Wertebereiche zugeordnet sind.

Gemäß einem weiteren Aspekt der Erfindung wird ein Beatmungsgerät vorgeschlagen, das das vorstehend beschriebene System zur Bestimmung einer regionalen Compliance umfasst, wobei das Beatmungsgerät beispielsweise zur druckunterstützenden Beatmung geeignet ist. Das System zur Bestimmung der regionalen Compliance kann erfindungsgemäß einen Teil des Beatmungsgeräts bilden und kann vorzugsweise auch für Funktionen genutzt werden, die über die Bestimmung der regionalen Compliance hinausgehen. Das Beatmungsgerät ist beispielsweise zur druckunterstützenden Beatmung geeignet. Somit kann es beispielsweise eine Lunge bei der Spontanatmung unterstützen. Dies kann gemäß möglichen Ausführungsformen erfolgen, indem von dem Beatmungsgerät ein Atemfluss überwacht wird und das Beatmungsgerät die Atemwege mit einem inspiratorischen Druck beaufschlagt, falls durch die Lunge kein ausreichender Atemfluss bewirkt wird.

Es kann erfindungsgemäß vorgesehen sein, dass das Beatmungsgerät dazu eingerichtet ist, das Inspirationsmanöver manuell, automatisch und/oder zyklisch auszulösen. Bei einer manuellen Auslösung löst das Beatmungsgerät das Inspirationsmanöver auf äußere Veranlassung hin aus, beispielsweise bei einer Betätigung eines Schalters durch einen Benutzer. In diesem Fall wird die Bestimmung der regionalen Compliance $C.REG_i$ durch den Benutzer veranlasst. Bei einer automatischen Auslösung wird das Inspirationsmanöver durch das Beatmungsgerät ausgelöst, wenn eine vordefinierte Bedingung erfüllt ist. Bei der vordefinierten Bedingung kann es sich beispielsweise um eine Atemfrequenz, einen durch den Benutzer unterschrittenen Atemdruck oder einen anderen Parameter physiologischer oder sonstiger Art handeln. Bei einer zyklischen Auslösung wird das Inspirationsmanöver in übereinstimmenden zeitlichen Abständen durch das Beatmungsgerät selbsttätig ausgelöst.

Nach einem weiteren Aspekt der Lehre ist das Beatmungsgerät dazu eingerichtet, in Abhängigkeit von mindestens einer regionalen Compliance einen Druck anzupassen, wobei der Druck insbesondere ein positiver endexspiratorischer Druck ist, ein Tidalvolumen anzupassen und/oder ein Rekrutierungsmanöver auszulösen. Das Beatmungsgerät kann ferner dazu eingerichtet sein, sonstige Behandlungsparameter anzupassen. Auf diese Weise können beispielsweise therapeutische Handlungen durch das Beatmungsgerät automatisch ausgelöst werden. Bei der mindestens einen Compliance kann es sich um die regionale Compliance $C.REG_i$ und/oder Compliances handeln, die das Beatmungsgerät für andere Lungenbereiche bestimmt hat. Die regionale Compliance bildet in vielen Fällen ein Beurteilungsmerkmal, das allein oder in Kombination mit anderen physiologischen Parametern herangezogen werden kann, um die vorbeschriebenen Handlungen auszulösen. Bei dem positiven endexspiratorischen Druck handelt es sich um einen Druck, mit dem das Beatmungsgerät die Atemwege am Ende der Exspiration beaufschlagt, um einen Kollaps von Lungenbläschen zu vermeiden. Bei dem Tidalvolumen handelt es sich um ein Atemzugvolumen. Das Beatmungsgerät kann dazu eingerichtet sein, ein gewisses Mindestatemzugvolumen sicherzustellen, und dieses lässt sich erfindungsgemäß in Abhängigkeit von der regionalen Compliance anpassen. Bei Rekrutierungsmanövern handelt es sich um Beatmungsmanöver, mit denen bezweckt wird, kollabierte Lungenbereiche zu belüften und/oder ein Kollabieren der Lunge oder von Teilbereichen der Lunge zu verhindern.

Es ist vorteilhaft, wenn das Beatmungsgerät ein Anzeigemittel aufweist und dazu eingerichtet ist, mehrere regionale Compliances in unterschiedlichen Lungenarealen graphisch darzustellen. Bei dem Anzeigemittel kann es sich erfindungsgemäß um einen Bildschirm oder dergleichen handeln. Gemäß einer besonderen Ausführungsform werden die regionalen Compliances anhand eines Abbilds einer Lunge graphisch dargestellt. Dabei können erfindungsgemäß Regionen der Lunge, in denen vergleichsweise hohe regionale Compliances vorliegen, mit einer anderen Farbgebung oder in einer anderen Helligkeit dargestellt werden als Regionen der Lunge, in denen vergleichsweise niedrige regionale Compliances vorliegen. Ein Betrachter kann somit eine örtliche Verteilung der regionalen Compliances in der Lunge auf einfache Weise nachvollziehen.

Die Erfindung betrifft auch eine Einrichtung zur Elektro-Impedanz-Tomographie umfassend ein System oder ausgebildet als Teil eines Systems, wobei die Einrichtung zur Elektro-Impedanz-Tomographie zumindest ein Anzeigemittel, zumindest eine Rechnereinheit und Mittel zur Bestimmung eines Lungendehnbarkeitswerts C.M durch eine Messung einer Compliance der Lunge oder durch eine Messung eines Surrogates für die Compliance der Lunge aufweist.

Weitere Aspekte der Erfindung werden anhand der Figuren beispielhaft erläutert. Dabei zeigt:

Fig. 1    eine Darstellung eines Druckverlaufs eines Atemwegsdrucks bei einem endinspiratorischen Holdmanöver und

Fig. 2    ein Punktdiagramm, das eine prozentuale Verteilung von regionalen Compliances in einer Lunge wiedergibt.

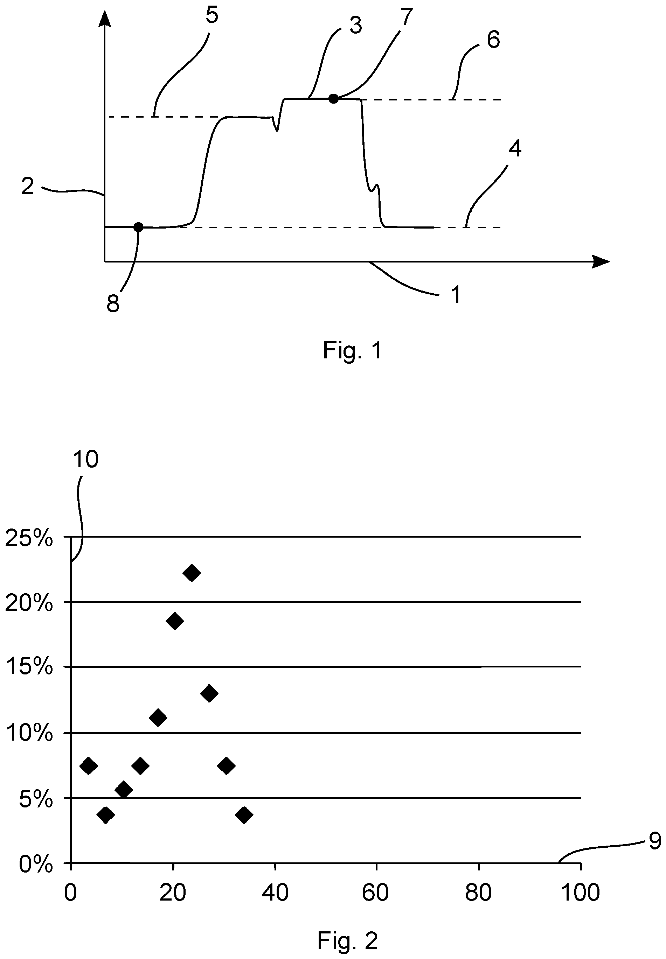

Fig. 1 zeigt eine Darstellung eines Druckverlaufs 3 eines Atemwegsdrucks bei einem endinspiratorischen Holdmanöver. Der Druckverlauf 3 ist über eine Zeitachse 1 und eine Druckachse 2 aufgetragen. Der dargestellte Druckverlauf zeigt einen typischen Verlauf der Druckänderung bei einem endinspiratorischen Holdmanöver unter druckunterstützter Spontanatmung. Zunächst liegt ein endexspiratorischer Druck 4 vor, dessen Niveau durch die untere gestrichelte Linie dargestellt wird.

Der endexspiratorische Druck 4 stellt sich am Ende der Exspiration ein. Es wird bevorzugt bei Okklusion der Atemwege gemessen, hierzu wird beispielhaft ein erster Messpunkt 8 aufgenommen.

Beim Einatmen steigt der Atemwegsdruck an, es ergibt sich zunächst ein endinspiratorischer Druck 5, dessen Niveau durch die in der Darstellung gemäß Fig. 1 obere linke, gestrichelte Linie dargestellt wird. Nun wird ein endinspiratorisches Holdmanöver ausgelöst, das heißt der Atemfluss wird unterbrochen, beispielsweise mittels eines Beatmungsgeräts. In diesem Fall stellt sich ein endinspiratorischer Plateaudruck 6 ein, der mittels der weiteren, in der Darstellung gemäß Fig. 1 oberen rechten, gestrichelten Linie dargestellt wird. Der endinspiratorische Plateaudruck 6 liegt bei druckunterstützender Beatmung in der Regel über dem endinspiratorischen Druck 5. Nun wird - bei anhaltender Okklusion der der Atemwege - der endinspiratorische Plateaudruck 6 gemessen, hier beispielhaft an einem zweiten Messpunkt 7. Nachdem die Okklusion der Atemwege aufhoben worden ist, kann eine Exspiration erfolgen, wobei der Atemwegsdruck abfällt und sich wieder der endexspiratorische Druck 4 einstellt.

Fig. 2 zeigt ein Punktdiagramm, das eine prozentuale Verteilung von regionalen Compliances in einer Lunge wiedergibt. Auf der X-Achse 9 ist eine regionale Compliance in der Einheit ml/mbar aufgetragen. Auf der Y-Achse 10 ist ein Anteil von EIT-Pixeln aufgetragen, denen die jeweilige regionale Compliance zuzuordnen ist. Die anteilsmäßige Verteilung der regionalen Compliance innerhalb der Lunge lässt sich somit nachvollziehen. Das Verfahren zur Berechnung der regionalen Compliances wird nachfolgend anhand von Beispielwerten erläutert, wozu beispielhaft auf die nachfolgende Tabelle 1 verwiesen wird:

**Tabelle 1**

| Impedanz-Wertebereich i | $W_i.UB$ | $NPX_i$ | $UNW_i$ | $CONTRIB_i$ |
|---|---|---|---|---|
| 1 | 5 | 40 | 0,250 | 10,000 |
| 2 | 10 | 20 | 0,500 | 10,000 |
| 3 | 15 | 30 | 0,750 | 22,500 |
| 4 | 20 | 40 | 1,000 | 40,000 |
| 5 | 25 | 60 | 1,250 | 75,000 |
| 6 | 30 | 100 | 1,500 | 150,000 |
| 7 | 35 | 120 | 1,750 | 210,000 |
| 8 | 40 | 70 | 2,000 | 140,000 |
| 9 | 45 | 40 | 2,250 | 90,000 |
| 10 | 50 | 20 | 2,500 | 50,000 |

Zugrunde gelegt wird eine Messung eines endexspiratorischen Drucks EEP und eines endinspiratorischen Plateaudrucks P.PLATEAU. Zu dem Zeitpunkt, zu dem der endexspiratorische Druck EEP gemessen wird, wird mittels Elektro-Impedanz-Tomographie ein Impedanzwert Z.EXSP in unterschiedlichen Lungenregionen gemessen. Zu dem Zeitpunkt, zu dem der endinspiratorische Plateaudruck P.PLATEAU gemessen wird, wird mittels Elektro-Impedanz-Tomographie eine elektrische Impedanz Z.INSP in unterschiedlichen Lungenregionen gemessen. Die gemessenen elektrischen Impedanzen sind unterschiedlichen EIT-Pixeln zugeordnet.

Aus Wertepaaren Z.EXSP und Z.INSP wird für jedes EIT-Pixel eine Impedanzdifferenz $\Delta Z = Z.INSP - Z.EXSP$ berechnet. Die Tabelle 1 führt Impedanz-Wertebereiche i auf. Die Impedanz-Wertebereiche sind jeweils gleich groß, sodass sich beispielsweise für einen ersten Impedanz-Wertebereich 1 eine Untergrenze $W_1.LB = 0$ und eine Obergrenze $W_1.UB = 5$ ergibt, hier als einheitenlose Größen angegeben. In der Tabelle sind Obergrenzen $W_i.UB$ der Impedanz-Wertebereiche angegeben. In einer angrenzenden Spalte ist für jeden Impedanz-Wertebereich eine Anzahl von EIT-Pixeln $NPX_i$ angegeben, die dem jeweiligen Wertebereich aufgrund ihrer Impedanzdifferenz $\Delta Z$ zuzuordnen sind. Für jeden Impedanz-Wertebereich wird ein ungewichteter Zwischenwert $UNW_i$ berechnet. Der ungewichtete Zwischenwert bestimmt sich gemäß der Formel:

$$UNW_i = W_i.UB / (P.PLATEAU - EEP)$$

Als Druckdifferenz zwischen P.PLATEAU und EEP wird im vorliegenden Beispiel 20 mbar angenommen. In einer angrenzenden Spalte wird ein Beitrag zur Compliance, berechnet gemäß der Formel:

$$CONTRIB_i = NPX_i * UNW_i$$

angegeben.

Die Berechnung wird in der Tabelle 2 fortgeführt. Es wird ein normalisierter Zwischenwert für den i-ten Impedanz-Wertebereich gemäß der Formel:

$$UNW\_NORM_i = UNW_i * C.M / \sum_{n=1}^{10} CONTRIB_n$$

berechnet. Dabei ist C.M eine Compliance der gesamten Lunge und beispielsweise gemäß der Formel:

$$C.M = \frac{TV}{P.PLATEAU - EEP}$$

aus einem gemessenen Tidalvolumen TV, dem gemessenen endinspiratorischen Plateaudruck P.PLATEAU und dem gemessenen endexspiratorischen Druck EEP berechnet. Vorliegend wird eine Compliance C.M = 20 ml/mbar angenommen.

**Tabelle 2**

| i | $UNW\_NORM_i$ | $C.REG_i$ [ml/mbar] | $A_i$ |
|---|---------------|---------------------|-------|
| 1 | 0,006 | 3,39 | 7% |
| 2 | 0,012 | 6,77 | 4% |
| 3 | 0,018 | 10,16 | 6% |
| 4 | 0,025 | 13,54 | 7% |
| 5 | 0,031 | 16,93 | 11% |
| 6 | 0,037 | 20,31 | 19% |
| 7 | 0,043 | 23,70 | 22% |
| 8 | 0,050 | 27,08 | 13% |
| 9 | 0,056 | 30,47 | 7% |
| 10 | 0,062 | 33,86 | 4% |

Schließlich wird eine regionale Compliance $C.REG_i$ gemäß der Formel:

$$C.REG_i = UNW\_NORM_i * NPX_i$$

berechnet. Legt man die beschriebene Berechnungsmethode zugrunde, so lässt sich daraus die vereinfachte Formel:

$$C.REG_i = \frac{W_i.UB}{\sum_{n=1}^{m}(NPX_n * W_n.UB)} * C.M * \sum_{n=1}^{m} NPX_n$$

ableiten. Um einen Anteil der EIT-Pixel zu bestimmen, bei denen die regionale Compliance $C.REG_i$ vorzufinden ist, wird ein Anteil des Lungenvolumens $A_i$ gemäß der Formel:

$$A_i = \frac{NPX_i}{\sum_{n=1}^{m} NPX_n}$$

berechnet. Die voranstehend ermittelten Werte $C.REG_i$ und $A_i$ werden in Fig. 2 visualisiert dargestellt. Aus einer solchen Visualisierung können Rückschlüsse auf den Zustand der Lunge gezogen werden und gegebenenfalls therapeutische Maßnahmen eingeleitet werden oder Beatmungsparameter angepasst werden.

**Bezugszeichenliste**

1.   Zeitachse
2.   Druckachse
3.   Druckverlauf
4.   Endexspiratorischer Druck
5.   Endinspiratorischer Druck
6.   Endinspiratorischer Plateaudruck
7.   Zweiter Messpunkt

8. Erster Messpunkt
9. X-Achse
10. Y-Achse

**Patentansprüche**

1. Verfahren zur Bestimmung einer regionalen Compliance $C.REG_i$ einer Lunge bei Spontanatmung, wobei folgende Schritte ausgeführt werden:

   - Bestimmung eines Lungendehnbarkeitswerts C.M durch eine Auslösung eines Inspirationsmanövers, sowie entweder durch eine Messung einer Compliance der gesamten Lunge oder durch eine Messung eines Surrogates für die Compliance der gesamten Lunge,
   - Erfassung einer elektrischen Impedanzverteilung entlang wenigstens eines zweidimensionalen Schnitts durch einen menschlichen Thorax mittels einer Einrichtung zur Elektro-Impedanz-Tomographie,
   - Unterteilung der erfassten elektrischen Impedanzverteilung zu verschiedenen Zeitpunkten in eine Vielzahl von EIT-Pixeln, **gekennzeichnet durch** die:
   - Bestimmung einer endexspiratorischen elektrischen Impedanz Z.EXSP jedes der EIT-Pixel sowie einer endinspiratorischen elektrischen Impedanz Z.INSP jedes der EIT-Pixel,
   - Bestimmung einer Impedanzdifferenz $\Delta Z$ jedes der EIT-Pixel, indem eine Differenz zwischen der endinspiratorischen elektrischen Impedanz Z.INSP und der endexspiratorischen elektrischen Impedanz Z.EXSP gebildet wird,
   - Bereitstellung mehrerer Impedanz-Wertebereiche und Zuordnung der EIT-Pixel zu den Impedanz-Wertebereichen in Abhängigkeit von der jeweiligen Impedanzdifferenz $\Delta Z$ der EIT-Pixel,
   - Bestimmung der regionalen Compliance $C.REG_i$ für spezifische EIT-Pixel, die einem Impedanz-Wertebereich i zugeordnet sind, in Abhängigkeit von einer Häufigkeitsverteilung aller EIT-Pixel auf die Impedanz-Wertebereiche und in Abhängigkeit von dem Lungendehnbarkeitswert C.M.

2. Verfahren nach Anspruch 1, **dadurch gekennzeichnet, dass** die Bestimmung der regionalen Compliance $C.REG_i$ bei einer druckunterstützenden Beatmung der Lunge erfolgt.

3. Verfahren nach einem der vorhergehenden Ansprüche, **dadurch gekennzeichnet, dass** das Inspirationsmanöver ein endinspiratorisches Holdmanöver ist, das bei der druckunterstützenden Beatmung der Lunge ausgeführt wird.

4. Verfahren nach einem der vorhergehenden Ansprüche, **dadurch gekennzeichnet, dass** der Lungendehnbarkeitswert C.M gemäß

$$C.M = \frac{TV}{P.PLATEAU - EEP}$$

bestimmt wird, wobei TV ein Tidalvolumen der Lunge ist, wobei P.PLATEAU ein endinspiratorischer Plateaudruck (5) ist, der sich bei dem endinspiratorischen Holdmanöver einstellt, und wobei EEP ein endexspiratorischer Druck (4) ist.

5. Verfahren nach einem der vorhergehenden Ansprüche, **dadurch gekennzeichnet, dass** die regionale Compliance $C.REG_i$ gemäß

$$C.REG_i = \frac{W_i.UB}{\sum_{n=1}^{m}(NPX_n * W_n.UB)} * C.M * \sum_{n=1}^{m} NPX_n$$

bestimmt wird, wobei die EIT-Pixel m Impedanz-Wertebereichen zugeordnet sind, wobei $NPX_n$ eine Anzahl von EIT-Pixeln ist, die einem n-ten Impedanz-Wertebereich der m Impedanz-Wertebereiche zugeordnet sind, wobei $W_i.UB$ eine Obergrenze des Impedanz-Wertebereichs i ist, und wobei $W_n.UB$ eine Obergrenze des n-ten Impedanz-Wertebereichs der m Impedanz-Wertebereiche ist.

**6.** Verfahren nach einem der vorhergehenden Ansprüche, **dadurch gekennzeichnet, dass** die Impedanz-Wertebereiche ermittelt werden, indem eine minimale ermittelte Impedanzdifferenz $\Delta Z_{min}$ von einer maximalen ermittelten Impedanzdifferenz $\Delta Z_{max}$ subtrahiert wird und ein sich ergebender Zahlenbereich in gleich große Abschnitte unterteilt wird, wobei die Abschnitte die Impedanz-Wertebereiche bilden.

**7.** Verfahren nach einem der vorhergehenden Ansprüche, **dadurch gekennzeichnet, dass** einem Impedanz-Wertebereich $W_n$ mit einer Untergrenze $W_n.LB$ und mit einer Obergrenze $W_n.UB$ ein EIT-Pixel mit dem Differenzwert $\Delta Z$ genau dann zugeordnet wird, wenn gilt $W_n.LB \le \Delta Z < W_n.UB$ oder $W_n.LB < \Delta Z \le W_n.UB$.

**8.** Verfahren nach einem der vorhergehenden Ansprüche, **dadurch gekennzeichnet, dass** ein Anteil $A_i$ eines Lungenvolumens, in dem die regionale Compliance $C.REG_i$ vorliegt, gemäß

$$A_i = \frac{NPX_i}{\sum_{n=1}^{m} NPX_n}$$

bestimmt wird, wobei die EIT-Pixel m Impedanz-Wertebereichen zugeordnet sind, wobei $NPX_i$ eine Anzahl der spezifischen EIT-Pixel ist und wobei $NPX_n$ eine Anzahl von EIT-Pixeln ist, die einem n-ten Impedanz-Wertebereich der m Impedanz-Wertebereiche zugeordnet sind.

**9.** System zur Bestimmung einer regionalen Compliance $C.REG_i$ einer Lunge bei Spontanatmung und/oder während der Beatmung, wobei das System eine Einrichtung zur Elektro-Impedanz-Tomographie und Mittel zur Bestimmung eines Lungendehnbarkeitswerts C.M durch eine Messung einer Compliance der Lunge oder durch eine Messung eines Surrogates für die Compliance der Lunge aufweist, und wobei das System zur Durchführung des Verfahrens nach einem der Ansprüche 1 bis 9 ausgebildet ist und wobei das System eine Einrichtung zur Elektro-Impedanz-Tomographie, ein Beatmungsgerät, zumindest ein Anzeigemittel, zumindest eine Rechnereinheit und Mittel zur Bestimmung eines Lungendehnbarkeitswerts C.M durch eine Messung einer Compliance der Lunge oder durch eine Messung eines Surrogates für die Compliance der Lunge aufweist.

**10.** System nach Anspruch 9, wobei das System dazu eingerichtet ist, einen Lungendehnbarkeitswert C.M durch eine Auslösung eines Inspirationsmanövers, sowie entweder durch eine Messung einer Compliance der gesamten Lunge oder durch eine Messung eines Surrogates für die Compliance der gesamten Lunge zu bestimmen, eine elektrische Impedanzverteilung entlang wenigstens eines zweidimensionalen Schnitts durch einen menschlichen Thorax mittels einer Einrichtung zur Elektro-Impedanz-Tomographie zu erfassen, die erfasste elektrische Impedanzverteilung zu verschiedenen Zeitpunkten in eine Vielzahl von EIT-Pixeln zu unterteilen, eine endexspiratorische elektrische Impedanz Z.EXSP jedes der EIT-Pixel sowie eine endinspiratorische elektrische Impedanz Z.INSP jedes der EIT-Pixel zu bestimmen, eine Impedanzdifferenz $\Delta Z$ jedes der EIT-Pixel zu bestimmen, indem es eine Differenz zwischen der endinspiratorischen elektrischen Impedanz Z.INSP und der endexspiratorischen elektrischen Impedanz Z.EXSP bildet, mehrere Impedanz-Wertebereiche bereitzustellen und die EIT-Pixel den Impedanz-Wertebereichen in Abhängigkeit von der jeweiligen Impedanzdifferenz $\Delta Z$ der EIT-Pixel zuzuordnen, und die regionale Compliance $C.REG_i$ für spezifische EIT-Pixel, die einem Impedanz-Wertebereich i zugeordnet sind, in Abhängigkeit von einer Häufigkeitsverteilung aller EIT-Pixel auf die Impedanz-Wertebereiche und in Abhängigkeit von dem Lungendehnbarkeitswert C.M zu bestimmen.

**11.** System nach zumindest einem der Anspruch 9 oder 10, **dadurch gekennzeichnet, dass** das System dazu eingerichtet ist, den Lungendehnbarkeitswert C.M gemäß

$$C.M = \frac{TV}{P.PLATEAU - EEP}$$

zu bestimmen, wobei TV ein Tidalvolumen der Lunge ist, wobei P.PLATEAU ein endinspiratorischer Plateaudruck ist, der sich nach der Auslösung eines endinspiratorischen Holdmanövers einstellt, und wobei EEP ein endexspiratorischer Druck ist.

**12.** System nach zumindest einem der vorhergehenden Ansprüche 9 bis 11, **dadurch gekennzeichnet, dass** das System dazu eingerichtet ist, die regionale Compliance $C.REG_i$ gemäß

$$C.REG_i = \frac{W_i.UB}{\sum_{n=1}^{m}(NPX_n * W_n.UB)} * C.M * \sum_{n=1}^{m} NPX_n$$

zu bestimmen, wobei die EIT-Pixel m Impedanz-Wertebereichen zugeordnet sind, wobei $NPX_n$ eine Anzahl von EIT-Pixeln ist, die einem n-ten Impedan Wertebereich der m Impedanz-Wertebereiche zugeordnet sind, wobei $W_i.UB$ eine Obergrenze des Impedanz-Wertebereichs i ist, und wobei $W_n.UB$ eine Obergrenze eines n-ten Impedanz-Wertebereichs der m Impedanz-Wertebereiche ist.

13. Beatmungsgerät umfassend ein System oder ausgebildet als Teil eines Systems nach Anspruch 9, wobei das Beatmungsgerät zur druckunterstützenden Beatmung geeignet ist.

14. Beatmungsgerät nach Anspruch 13, **dadurch gekennzeichnet, dass** das Beatmungsgerät dazu eingerichtet ist, das Inspirationsmanöver manuell, automatisch und/oder zyklisch auszulösen.

15. Beatmungsgerät nach zumindest einem der vorhergehenden Ansprüche 13 bis 14, **dadurch gekennzeichnet, dass** das Beatmungsgerät dazu eingerichtet ist, in Abhängigkeit von mindestens einer regionalen Compliance einen Druck anzupassen, wobei der Druck insbesondere ein positiver endexspiratorischer Druck (4) ist, ein Tidalvolumen anzupassen und/oder ein Rekrutierungsmanöver auszulösen.

16. Beatmungsgerät nach zumindest einem der vorhergehenden Ansprüche 13 bis 15, **dadurch gekennzeichnet, dass** das Beatmungsgerät ein Anzeigemittel aufweist und dazu eingerichtet ist, mehrere regionale Compliances in unterschiedlichen Lungenarealen graphisch darzustellen.

17. Beatmungsgerät nach zumindest einem der vorhergehenden Ansprüche 13 bis 16, **dadurch gekennzeichnet, dass** auf dem Anzeigemittel eine graphische Repräsentation der Lunge dargestellt ist und in der graphischen Repräsentation der Lunge zumindest zwei regionale Compliances, in graphisch unterschiedlicher Darstellung, dargestellt sind.

18. Beatmungsgerät nach zumindest einem der vorhergehenden Ansprüche 13 bis 17, **dadurch gekennzeichnet, dass** auf dem Anzeigemittel zumindest zwei regionale Compliances als Prozentwert oder in ml/mbar dargestellt sind.

19. Einrichtung zur Elektro-Impedanz-Tomographie umfassend ein System oder ausgebildet als Teil eines Systems nach Anspruch 9, wobei die Einrichtung zur Elektro-Impedanz-Tomographie zumindest ein Anzeigemittel, zumindest eine Rechnereinheit und Mittel zur Bestimmung eines Lungendehnbarkeitswerts C.M durch eine Messung einer Compliance der Lunge oder durch eine Messung eines Surrogates für die Compliance der Lunge aufweist.

**Claims**

1. A method for determining regional compliance $C.REG_i$ of a lung in spontaneous breathing, wherein the following steps are performed:

 - determining a lung extensibility value C.M by triggering an inspiratory maneuver, as well as either by measuring a compliance of the entire lung or by measuring a surrogate for the compliance of the entire lung,
 - detecting an electrical impedance distribution along at least one two-dimensional section through a human thorax by means of an apparatus for electrical impedance tomography,
 - dividing the detected electrical impedance distribution at various points in time into a plurality of BIT pixels, **characterized by**:
 - determining an end-expiratory electrical impedance Z.EXSP of each of the EIT pixels and an end-inspiratory electrical impedance Z.INSP of each of the EIT pixels,
 - determining an impedance difference $\Delta Z$ of each of the EIT pixels by forming a difference between the end-inspiratory electrical impedance Z.INSP and the end-expiratory electrical impedance Z.EXSP,
 - providing multiple impedance value ranges and assigning the EIT pixels to the impedance value ranges as a function of the respective impedance difference $\Delta Z$ of the EIT pixels,
 - determining the regional compliance $C.REG_i$ for specific EIT pixels that are assigned to an impedance value range i, as a function of a frequency distribution of all EIT pixels over the impedance value ranges and as a function of the lung extensibility value C.M.

2.  The method according to claim 1, **characterized in that** the determination of the regional compliance $C.REG_i$ takes place during pressure-assisted ventilation of the lung.

3.  The method according to any one of the preceding claims, **characterized in that** the inspiratory maneuver is an end-inspiratory hold maneuver that is performed during pressure-assisted ventilation of the lung.

4.  The method according to any one of the preceding claims, **characterized in that** the lung extensibility value C.M is determined according to

$$C.M = \frac{TV}{P.PLATEAU - EEP}$$

wherein TV is a tidal volume of the lung, wherein P.PLATEAU is an end-inspiratory plateau pressure (5) that arises during the end-inspiratory hold maneuver, and wherein EEP is an end-expiratory pressure (4).

5.  The method according to any one of the preceding claims, **characterized in that** the regional compliance $C.REG_i$ is determined according to

$$C.REG_i = \frac{W_i.UB}{\sum_{n=1}^{m}(NPX_n * W_n.UB)} * C.M * \sum_{n=1}^{m} NPX_n$$

wherein the EIT pixels are assigned to m impedance value ranges, wherein $NPX_n$ is a number of EIT pixels that are assigned to an nth impedance value range of the m impedance value ranges, wherein $W_i.UB$ is an upper limit of the impedance value range i, and wherein $W_n.UB$ is an upper limit of the nth impedance value range of the m impedance value ranges.

6.  The method according to any one of the preceding claims, **characterized in that** the impedance value ranges are ascertained by subtracting a minimum ascertained impedance difference $\Delta Z_{min}$ from a maximum ascertained impedance difference $\Delta Z_{max}$ and by dividing a resulting numerical range into sections of equal size, wherein the sections form the impedance value ranges.

7.  The method according to any one of the preceding claims, **characterized in that** an impedance value range $W_n$ with a lower limit $W_n.LB$ and an upper limit $W_n.UB$ is assigned an EIT pixel with the difference value $\Delta Z$ exactly when $W_n.LB \leq \Delta Z < W_n.UB$ or $W_n.LB < \Delta Z \leq W_n.UB$.

8.  The method according to any one of the preceding claims, **characterized in that** a proportion $A_i$ of a lung volume in which the regional compliance $C.REG_i$ is present is determined according to

$$A_i = \frac{NPX_i}{\sum_{n=1}^{m} NPX_n}$$

wherein the EIT pixels are assigned m impedance value ranges, wherein $NPX_i$ is a number of the specific EIT pixels and wherein $NPX_n$ is a number of EIT pixels that are assigned to an nth impedance value range of the m impedance value ranges.

9.  A system for determining regional compliance $C.REG_i$ of a lung in spontaneous breathing and/or during ventilation, wherein the system comprises an apparatus for electrical impedance tomography and means for determining a lung extensibility value C.M by measuring a compliance of the lung or by measuring a surrogate for the compliance of the lung, and wherein the system is designed to carry out the method according to any one of claims 1 to 9 and wherein the system comprises an apparatus for electrical impedance tomography, a ventilation device, at least one display means, at least one computer unit, and means for determining a lung extensibility value C.M by measuring a compliance of the lung or by measuring a surrogate for the compliance of the lung.

10. The system according to claim 9, wherein the system is configured to determine a lung extensibility value C.M by triggering an inspiratory maneuver, as well as either by measuring a compliance of the entire lung or by measuring a surrogate for the compliance of the entire lung, detecting an electrical impedance distribution along at least one two-dimensional section through a human thorax by means of an apparatus for electrical impedance tomography, dividing the detected electrical impedance distribution at various points in time into a plurality of EIT pixels, determining an end-expiratory electrical impedance Z.EXSP of each of the EIT pixels as well as an end-inspiratory electrical impedance Z.INSP of each of the EIT pixels, determining an impedance difference $\Delta Z$ of each of the EIT pixels by forming a difference between the end-inspiratory electrical impedance Z.INSP and the end-expiratory electrical impedance Z.EXSP, providing multiple impedance value ranges and assigning the EIT pixels to the impedance value ranges as a function of the respective impedance difference $\Delta Z$ of the EIT pixels, and determining the regional compliance $C.REG_i$ for specific EIT pixels that are assigned to an impedance value range i, as a function of a frequency distribution of all EIT pixels over the impedance value ranges and as a function of the lung extensibility value C.M.

11. The system according to at least one of claims 9 or 10, **characterized in that** the system is configured to determine the lung extensibility value C.M according to

$$C.M = \frac{TV}{P.PLATEAU - EEP}$$

wherein TV is a tidal volume of the lung, wherein P.PLATEAU is an end-inspiratory plateau pressure that arises after an end-inspiratory hold maneuver has been triggered, and wherein EEP is an end-expiratory pressure.

12. The system according to at least one of claims 9 to 11, **characterized in that** the system is configured to determine the regional compliance $C.REG_i$ according to

$$C.REG_i = \frac{W_i.UB}{\sum_{n=1}^{m}(NPX_n * W_n.UB)} * C.M * \sum_{n=1}^{m} NPX_n$$

wherein the EIT pixels are assigned to m impedance value ranges, wherein $NPX_n$ is a number of EIT pixels that are assigned to an nth impedance value range of the m impedance value ranges, wherein $W_i.UB$ is an upper limit of the impedance value range i, and wherein $W_n.UB$ is an upper limit of an nth impedance value range of the m impedance value ranges.

13. A ventilation device comprising a system or designed as part of a system according to claim 9, wherein the ventilation device is suitable for pressure-assisted ventilation.

14. The ventilation device according to claim 13, **characterized in that** the ventilation device is configured to trigger the inspiratory maneuver manually, automatically, and/or cyclically.

15. The ventilation device according to at least one of claims 13 to 14, **characterized in that** the ventilation device is configured to adapt a pressure as a function of at least one regional compliance, wherein the pressure is, in particular, a positive end-expiratory pressure (4), to adapt a tidal volume, and/or to trigger a recruitment maneuver.

16. The ventilation device according to at least one of the preceding claims 13 to 15, **characterized in that** the ventilation device comprises a display means and is configured to graphically present multiple regional compliances in different lung areas.

17. The ventilation device according to at least one of the preceding claims 13 to 16, **characterized in that** a graphical representation of the lung is presented on the display means and at least two regional compliances, graphically presented differently, are presented in the graphical representation of the lung.

18. The ventilation device according to at least one of the preceding claims 13 to 17, **characterized in that** at least two regional compliances are presented on the display means as a percentage value or in ml/mbar.

19. An apparatus for electrical impedance tomography comprising a system or designed as part of a system according to claim 9, wherein the apparatus for electrical impedance tomography comprises at least one display means, at least one computer unit, and means for determining a lung extensibility value C.M by measuring a compliance of the lung or by measuring a surrogate for the compliance of the lung.

# EP 3 861 932 B1

**Revendications**

1. Procédé de détermination d'une compliance régionale C.REG$_i$ d'un poumon pendant la respiration spontanée, les étapes suivantes étant exécutées :

   - détermination d'une valeur de compliance pulmonaire C.M par un déclenchement d'une manoeuvre d'inspiration, ainsi que par une mesure d'une compliance du poumon entier ou par une mesure d'un substitut pour la compliance du poumon entier,
   - saisie d'une distribution d'impédance électrique le long d'au moins une coupe bidimensionnelle par un thorax humain au moyen d'un dispositif de tomographie par impédance électrique,
   - subdivision de la distribution d'impédance électrique saisie à différents instants en une multiplicité de pixels EIT, **caractérisée par** la :
   - détermination d'une impédance électrique de fin d'expiration Z.EXSP de chacun des pixels EIT ainsi que d'une impédance électrique de fin d'inspiration Z.INSP de chacun des pixels EIT,
   - détermination d'une différence d'impédance $\Delta Z$ de chacun des pixels EIT par la formation d'une différence entre l'impédance électrique de fin d'inspiration Z.INSP et l'impédance électrique de fin d'expiration Z.EXSP,
   - mise à disposition de plusieurs plages de valeurs d'impédance et attribution des pixels EIT aux plages de valeurs d'impédance en fonction de la différence d'impédance respective $\Delta Z$ des pixels EIT,
   - détermination de la compliance régionale C.REG$_i$ pour des pixels EIT spécifiques, lesquels sont attribués à une plage de valeurs d'impédance i, en fonction d'une distribution des fréquences de tous les pixels EIT sur les plages de valeurs d'impédance et en fonction de la valeur de compliance pulmonaire C.M.

2. Procédé selon la revendication 1, **caractérisé en ce que** la détermination de la compliance régionale C.REG$_i$ est effectuée lors d'une ventilation du poumon avec assistance par pression.

3. Procédé selon l'une des revendications précédentes, **caractérisé en ce que** la manoeuvre d'inspiration est une manoeuvre de pause de fin d'inspiration qui est effectuée lors de la ventilation du poumon avec assistance par pression.

4. Procédé selon l'une des revendications précédentes, **caractérisé en ce que** la valeur de compliance pulmonaire C.M est déterminée conformément à

$$C.M = \frac{TV}{P.PLATEAU - EEP}$$

   TV étant un volume courant du poumon, P.PLATEAU étant une pression de plateau de fin d'inspiration (5), laquelle se produit lors de la manoeuvre de pause de fin d'inspiration, et EEP étant une pression de fin d'expiration (4).

5. Procédé selon l'une des revendications précédentes, **caractérisé en ce que** la compliance régionale C.REG$_i$ est déterminée conformément à

$$C.REG_i = \frac{W_i.UB}{\sum_{n=1}^{m}(NPX_n * W_n.UB)} * C.M * \sum_{n=1}^{m} NPX_n$$

   les pixels EIT étant attribués à m plages de valeurs d'impédance, NPX$_n$ étant un nombre de pixels EIT attribués à une n-ième plage de valeurs d'impédance des m plages de valeurs d'impédance, W$_i$.UB étant une limite supérieure de la plage de valeurs d'impédance i et W$_n$.UB étant une limite supérieure de la n-ième plage de valeurs d'impédance des m plages de valeurs d'impédance.

6. Procédé selon l'une des revendications précédentes, **caractérisé en ce que** les plages de valeurs d'impédance sont déterminées en soustrayant une différence d'impédance déterminée minimale $\Delta Z_{min}$ d'une différence d'impédance déterminée maximale $\Delta Z_{max}$ et en divisant en sections de même taille une plage de nombres résultante, les sections formant les plages de valeurs d'impédance.

7. Procédé selon l'une des revendications précédentes, **caractérisé en ce qu** un pixel EIT avec la valeur de différence $\Delta Z$ est attribué à une plage de valeurs d'impédance W$_n$ avec une limite inférieure W$_n$.LB et avec une limite supérieure W$_n$.UB exactement lorsque W$_n$.LB $\leq \Delta Z <$ W$_n$.UB ou W$_n$.LB $< \Delta Z \leq$ W$_n$.UB s'applique

**8.** Procédé selon l'une des revendications précédentes, **caractérisé en ce qu'**une part $A_i$ d'une capacité pulmonaire dans laquelle la compliance régionale C.REG$_i$ se trouve est déterminée conformément à

$$A_i = \frac{NPX_i}{\sum_{n=1}^{m} NPX_n}$$

les pixels EIT étant attribués à m plages de valeurs d'impédance, NPX$_i$ étant un nombre des pixels EIT spécifiques et NPX$_n$ étant un nombre de pixels EIT attribués à une n-ième plage de valeurs d'impédance des m plages de valeurs d'impédance.

**9.** Système de détermination d'une compliance régionale C.REG$_i$ d'un poumon pendant la respiration spontanée et/ou pendant la ventilation, le système présentant un dispositif de tomographie par impédance électrique et des moyens de détermination d'une valeur de compliance pulmonaire C.M par une mesure d'une compliance du poumon ou par une mesure d'un substitut pour la compliance du poumon, et le système pour l'exécution du procédé étant conçu selon l'une des revendications 1 à 9 et le système présentant un dispositif de tomographie par impédance électrique, un appareil respiratoire, au moins un moyen d'affichage, au moins une unité de calcul et des moyens pour la détermination d'une valeur de compliance pulmonaire C.M par une mesure d'une compliance du poumon ou par une mesure d'un substitut de la compliance du poumon.

**10.** Système selon la revendication 9, le système étant agencé pour déterminer une valeur de compliance pulmonaire C.M par un déclenchement d'une manoeuvre d'inspiration, ainsi que par une mesure d'une compliance du poumon entier ou par une mesure d'un substitut pour la compliance du poumon entier, pour saisir une répartition d'impédance électrique le long d'au moins une coupe bidimensionnelle par un thorax humain au moyen d'un dispositif de tomographie par impédance électrique, pour subdiviser la répartition d'impédance électrique saisie à différents instants en une multiplicité de pixels EIT, pour déterminer une impédance électrique de fin d'expiration Z.EXSP de chacun des pixels EIT ainsi qu'une une impédance électrique de fin d'inspiration Z.INSP de chacun des pixels EIT, pour déterminer une différence d'impédance ΔZ de chacun des pixels EIT en formant une différence entre l'impédance électrique de fin d'inspiration Z.INSP et l'impédance électrique de fin d'expiration Z.EXSP, pour fournir plusieurs plages de valeurs d'impédance et attribuer les pixels EIT aux plages de valeurs d'impédance en fonction de la différence d'impédance respective ΔZ des pixels EIT, et pour déterminer la compliance régionale C.REG$_i$ pour des pixels EIT spécifiques qui sont attribués à une plage de valeurs d'impédance i en fonction d'une distribution des fréquences de tous les pixels EIT sur les plages de valeurs d'impédance et en fonction de la valeur de compliance pulmonaire C.M.

**11.** Système selon au moins l'une des revendications 9 ou 10, **caractérisé en ce que** le système est agencé pour déterminer la valeur de compliance pulmonaire C.M conformément à

$$C.M = \frac{TV}{P.PLATEAU - EEP}$$

TV étant un volume courant du poumon, P.PLATEAU étant une pression de plateau de fin d'inspiration, laquelle se produit après le déclenchement d'une manoeuvre de pause de fin d'inspiration, et EEP étant une pression de fin d'expiration.

**12.** Système selon au moins l'une des revendications précédentes 9 à 11, **caractérisé en ce que** le système est agencé pour déterminer la compliance régionale C.REG$_i$ conformément à

$$C.REG_i = \frac{W_i.UB}{\sum_{n=1}^{m} (NPX_n * W_n.UB)} * C.M * \sum_{n=1}^{m} NPX_n$$

les pixels EIT étant attribués à m plages de valeurs d'impédance, NPX$_n$ étant un nombre de pixels EIT attribués à une n-ième plage de valeurs d'impédance des m plages de valeurs d'impédance, W$_i$.UB étant une limite supérieure de la plage de valeurs d'impédance i et W$_n$.UB étant une limite supérieure de la n-ième plage de valeurs d'impédance des m plages de valeurs d'impédance.

**13.** Appareil respiratoire comprenant un système ou conçu comme partie d'un système selon la revendication 9, l'appareil respiratoire étant adapté à la ventilation avec assistance par pression.

14. Appareil respiratoire selon la revendication 13, **caractérisé en ce que** l'appareil respiratoire est agencé pour déclencher la manoeuvre d'inspiration de façon manuelle, automatique et/ou cyclique.

15. Appareil respiratoire selon au moins l'une des revendications précédentes 13 à 14, **caractérisé en ce que** l'appareil respiratoire est agencé pour ajuster une pression en fonction d'au moins une compliance régionale, la pression étant en particulier une pression de fin d'expiration positive (4), pour ajuster un volume courant et/ou pour déclencher une manoeuvre de recrutement.

16. Appareil respiratoire selon au moins l'une des revendications précédentes 13 à 15, **caractérisé en ce que** l'appareil respiratoire présente un moyen d'affichage et est agencé pour représenter graphiquement plusieurs compliances régionales dans différentes zones pulmonaires.

17. Appareil respiratoire selon au moins l'une des revendications précédentes 13 à 16, **caractérisé en ce qu'**une représentation graphique du poumon est représentée sur le moyen d'affichage et **en ce qu'**au moins deux compliances régionales sont représentées sur la représentation graphique du poumon, par des représentations graphiquement différentes.

18. Appareil respiratoire selon au moins l'une des revendications précédentes 13 à 17, **caractérisé en ce qu'**au moins deux compliances régionales sont représentées sur le moyen d'affichage en valeur de pourcentage ou en ml/mbar.

19. Dispositif de tomographie par impédance électrique comprenant un système ou conçu comme partie d'un système selon la revendication 9, l'appareil de tomographie par impédance électrique comprenant au moins un moyen d'affichage, au moins une unité de calcul et des moyens pour la détermination d'une valeur de compliance pulmonaire C.M par une mesure d'une compliance du poumon ou par une mesure d'un substitut pour la compliance du poumon.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102013203177 A1

**In der Beschreibung aufgeführte Nicht-Patentliteratur**

- **FOTI et al.** End-inspiratory airway occlusion: a method to assess the pressure developed by inspiratory muscles in patients with acute lung injury undergoing pressure support. *American Journal of Respiratory and Critical Care Medicine,* 1997, vol. 156 (4), 1210-1216

- **BELLANI et al.** Driving Pressure Is Associated with Outcome during Assisted Ventilation in Acute Respiratory Distress Syndrome. *Anesthesiology,* 2019, vol. 131 (3), 594-604